# EUROPEAN PATENT APPLICATION

(11) **EP 2 910 235 A1**
(43) Date of publication of application: **26.08.2015**
(21) Application number: 13846834.3
(22) Date of filing: 15.10.2013
(51) Int. Cl.: A61J 3/00, A61M 39/00

(54) **PUNCTURE NEEDLE ADAPTER**

(30) Priority: 16.10.2012 JP 2012228712
(71) Applicant: JMS Co., Ltd., Hiroshima-shi, Hiroshima 730-8652 (JP)
(72) Inventor: TAKEUCHI, Masahiko, Naka-ku, Hiroshima-shi Hiroshima 730-8652 (JP)
(74) Representative: Piésold, Alexander James
(86) International application number: PCT/JP2013/077969
(87) International publication number: WO 2014/061661

(57) **Abstract**

A puncture needle (10) protrudes from the center of an approximately cross-shaped top plate (20) from which four arms (21) are protruded. From the tip of each of the arms, two elastic pieces (22) divided by a slit (23) extend toward the same side as the puncture needle. The tips of the two elastic pieces extending respectively from two adjacent arms are joined via a bridging portion (24) so as to form an approximately U-shaped holding piece (25). An engaging claw (30) protrudes toward the puncture needle from the bridging portion of the holding piece. The engaging claw can be displaced in a direction away from the puncture needle by elastic deformation of the elastic pieces.

## Description

### Technical Field

The present invention relates to a puncture needle adapter that includes an engaging claw for stably keeping a state where a puncture needle is stuck into a female connector.

### Background Art

In a case of administering a drug solution to a patient through a vein, a female connector (port) of a drug solution container that stores a prepared drug solution and the vein of the patient are connected to each other via an administration set including a flexible tube. Typically, to the female connector of the drug solution container, a rubber plug is provided. This rubber plug is punctured with a puncture needle (which is called also as "bottle needle") that has a sharp tip and that is provided at the upstream end of the administration set, so that the drug solution container and the administration set are brought into communication with each other.

If the puncture needle is simply stuck into the rubber plug, there is a chance that the puncture needle unintentionally comes out of the rubber plug when an external force or vibration is applied to the female connector or to the puncture needle. As a result, the drug solution may leak out to the outside.

There are cases where the drug solution contains a drug designated as a dangerous drug, such as some anticancer drugs for example. Therefore, it is necessary to avoid a situation where such a dangerous drug solution leaks out and adheres to the operator's finger or the like, or the operator inhales the vapor from the solution.

For the purpose of preventing the puncture needle from unintentionally coming out of the rubber plug, various types of puncture needle adapters each having a puncture needle provided integrally with an engaging structure to be engaged with a female connector have been proposed.

For example, Patent document 1 describes a puncture needle adapter having four engaging hook elements provided upright on a housing so as to surround a puncture needle protruding from the center of the housing. At the tip of each of the engaging hook elements, a hook protrusion protruding toward the puncture needle is provided. The hook protrusion engages with the flange of the female connector, thereby preventing the puncture needle from coming out of the rubber plug.

Patent document 2 describes a puncture needle adapter equipped with a shroud having an approximately cylindrical shape to surround a puncture needle. On the inner circumferential face of the shroud, a pair of first claws that are comparatively short and have high rigidity and a pair of second claws that are comparatively long and plastically deformable protrude toward the puncture needle. In a case of connecting the puncture needle adapter to a female connector including a flange having a relatively large outer diameter, the second claws are deformed and the first claws engage with the flange of the female connector. In a case of connecting the puncture needle adapter to a female connector including a flange having a relatively small outer diameter, the second claws engage with the flange of the female connector. In this manner, any of the first claws and the second claws engage with the flange depending on the size of the flange of the female connector, thereby preventing the puncture needle from coming out of the rubber plug.

### Prior Art Documents

### Patent documents

Patent document 1: JP 2012-511940
Patent document 2: JP 4509569

### Disclosure of Invention

### Problem to be Solved by the Invention

Regarding the puncture needle adapter as described in the Patent document 1 above, since the movable range of the engaging hook elements is small, there is a limit on the size of the connectable flange. For example, it is difficult to engage the hook protrusions of the engaging hook elements with a flange having a large outer diameter.

Since the puncture needle adapter described in Patent document 2 above includes two types of claws, the range in size of the connectable flange is wider in comparison with the puncture needle adapter of Patent document 1. However, the two types of claws respectively have the optimal ranges for the size of flange, and thus it is difficult to engage any of the two types of claws with a flange having a size out of the ranges. Furthermore, since the shroud having an approximately cylindrical shape surrounds the periphery of the puncture needle, in a case of sticking the puncture needle into the rubber plug of the female connector, it is difficult to observe from outside the state of the puncture needle stuck into the rubber plug.

The present invention relates to a puncture needle adapter including an engaging claw to be engaged with a female connector. A first object of the present invention is to enlarge the range of the size of the connectable female connector in the puncture needle adapter. A second object of the present invention is to enable to observe visually the connected state of the puncture needle and the female connector in the puncture needle adapter.

### Means for Solving Problem

A puncture needle adapter of the present invention comprises a puncture needle, and an engaging claw to be engaged with a female connector when the puncture needle is stuck into the female connector. The puncture needle adapter comprises further a top plate from which four arms protrude to form an approximately cross shape. The puncture needle protrudes from the center of the top plate. Two elastic pieces divided by a slit extend from the tip of each of the four arms toward the same side as the puncture needle so as to oppose the puncture needle. The tips of two of the elastic pieces extending respectively from two adjacent arms are joined with each other via a bridging portion so as to form an approximately U-shaped holding piece. The engaging claw is formed at the bridging portion of the holding piece so as to protrude toward the puncture needle. The engaging claw can be displaced in a direction away from the puncture needle by elastic deformation of the two elastic pieces constituting the holding piece.

### Effects of the Invention

The puncture needle adapter of the present invention comprises an engaging claw to be engaged with a female connector when the puncture needle is stuck into the female connector. Accordingly, there is little chance that the puncture needle stuck into the female connector unintentionally comes out of the female connector due to an external force, vibration or the like.

An approximately U-shaped holding piece provided with an engaging claw is provided to adjacent arms so as to bridge these arms. Accordingly, the two elastic pieces constituting the holding piece can undergo elastic bending deformation considerably. As a result, the range in size of a connectable female connector is increased.

A large opening is formed each by two arms protruding from the top plate and an approximately U-shaped holding piece joined with the two arms. Through the opening, it is possible to observe visually the puncture needle and the process that the puncture needle is stuck into the female connector.

### Brief Description of Drawings

[FIG. 1A] FIG. 1A is a perspective view showing a puncture needle adapter observed from above according to one embodiment of the present invention.
[FIG. 1B] FIG. 1B is a perspective view showing a puncture needle adapter observed from below according to one embodiment of the present invention.
[FIG. 2A] FIG. 2A is a cross-sectional view showing the puncture needle adapter according to one embodiment of the present invention, taken along a plane in the up-down direction including the line 2A-2A in FIG. 1A.
[FIG. 2B] FIG. 2B is a cross-sectional view showing the puncture needle adapter according to one embodiment of the present invention, taken along a plane in the up-down direction including the line 2B-2B in FIG. 1A.
[FIG. 3] FIG. 3 is a perspective view showing a puncture needle adapter according to one embodiment of the present invention, just before being connected to a female connector.
[FIG. 4A] FIG. 4A is a perspective view showing a puncture needle adapter observed from above according to one embodiment of the present invention, in a state being connected to a female connector.
[FIG. 4B] FIG. 4B is a perspective view showing a puncture needle adapter observed from below according to one embodiment of the present invention, in a state being connected to a female connector.
[FIG. 5] FIG. 5 is a cross-sectional view showing the puncture needle adapter according to one embodiment of the present invention, taken along a plane in the up-down direction including the line 5-5 in FIG. 4A, in a state being connected to a female connector.

### Description of the Invention

In the above-described puncture needle adapter of the present invention, it is preferable that an engaging face of the engaging claw to oppose the top plate is inclined to approach the top plate with decreasing distance from the puncture needle. According to the preferred constitution, even if the female connector has a large outer diameter, the chance of unintentional disengagement between the female connector and the engaging claw is decreased. Accordingly, the chance that the puncture needle unintentionally comes out of the female connector is decreased further.

It is preferable that the elastic pieces are inclined to be separated from the puncture needle with increasing distance from the top plate. According to the preferred constitution, it is possible to bend and deform the elastic pieces easily with the pressing force applied to press the puncture needle adapter into the female connector at the time of connecting the puncture needle adapter to the female connector, and thus the pressing force can be decreased.

It is preferable that the slit reaches the tip of the arm or the vicinity. According to the preferred constitution, it is possible to enlarge the range for allowing elastic bending deformation of the elastic pieces without sacrificing the strength of the arm, and thus it is possible to further enlarge the range in the size of the connectable female connector.

It is preferable that an edge of the bridging portion of the holding piece at the side opposite to the top plate is formed of a smooth curve. According to the preferred constitution, the chance that the operator feels pain or injures his/her finger by touching the edge of the bridging portion with his/her finger is decreased.

It is preferable that the engaging claw comprises a slidable face at the side opposite to the top plate, and the slidable face is inclined to approach the top plate with decreasing distance from the puncture needle. According to the preferred constitution, it is possible to engage the engaging claw with the female connector by only pressing the puncture needle toward the female connector. Accordingly, the operations for connection to the female connector can be simplified.

It is preferable that a self-closing connector communicating with the puncture needle is provided on a surface of the top plate opposite to the puncture needle. According to the preferred constitution, the chance that the drug solution leaks out to the outside is decreased in a condition where the puncture needle adapter and the administration set for example are not connected to each other.

The present invention will be described below in detail while disclosing preferred embodiments. However, it goes without saying that the present invention is not limited to the following embodiments. For the sake of convenience in the description, the drawings that are referenced in the following description show simplifications of, among the constituent members of the embodiment of the present invention, only relevant members that are necessary for describing the present invention. The present invention therefore can include arbitrary constituent members that are not shown in the following drawings. Also, regarding the dimensions of the members in the drawings, the dimensions of the actual constituent members, the ratios of the dimensions of the members, and the like are not shown faithfully

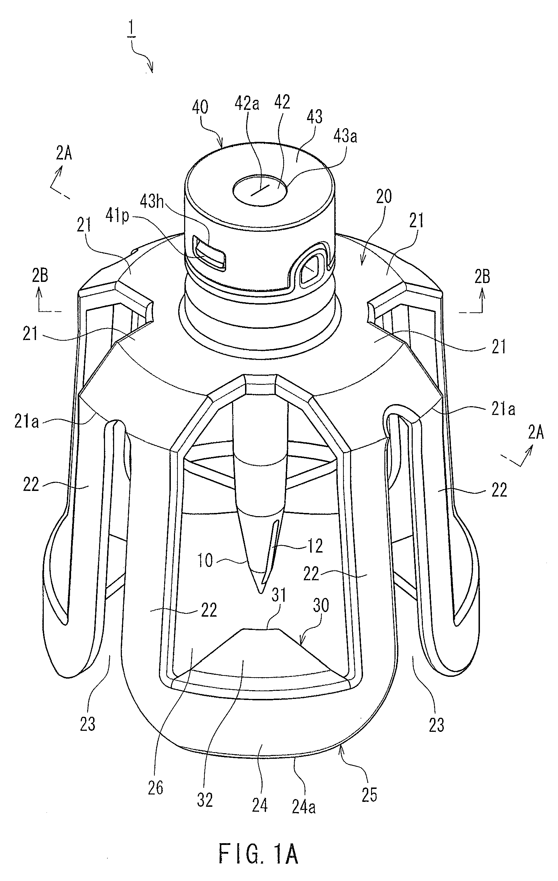

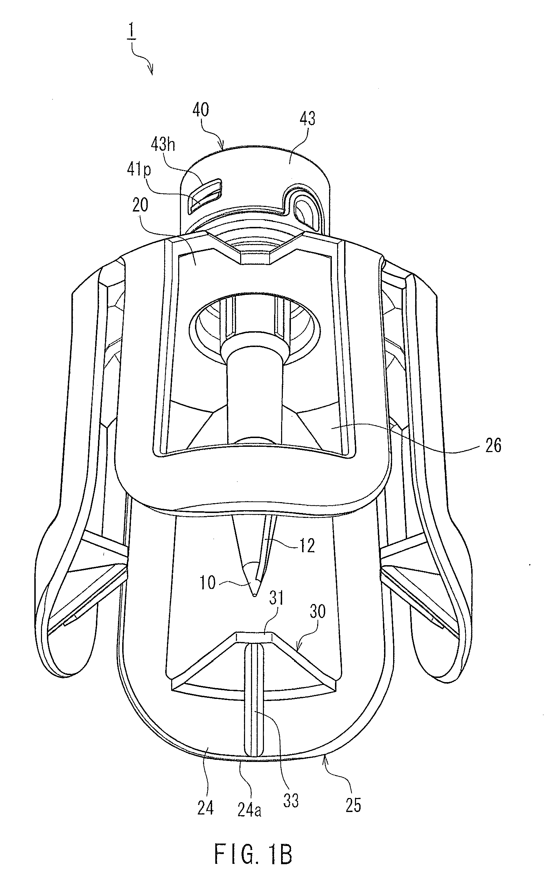

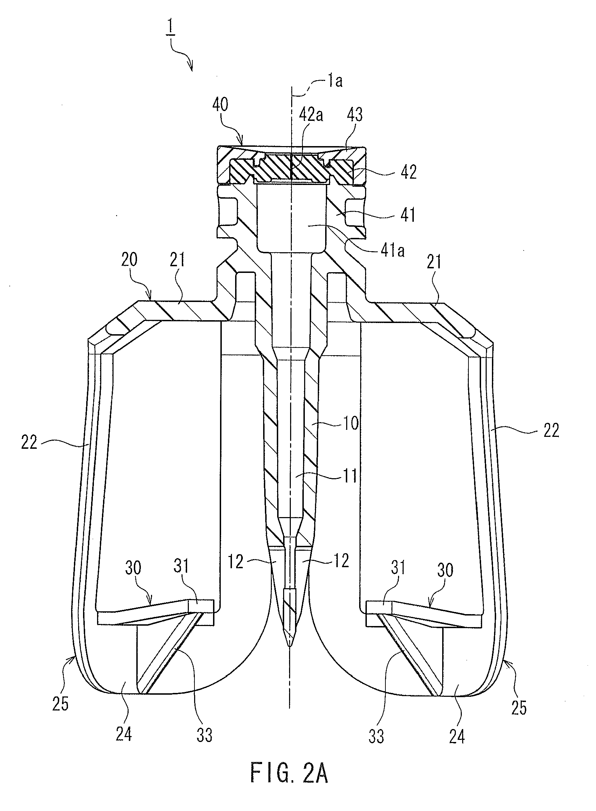

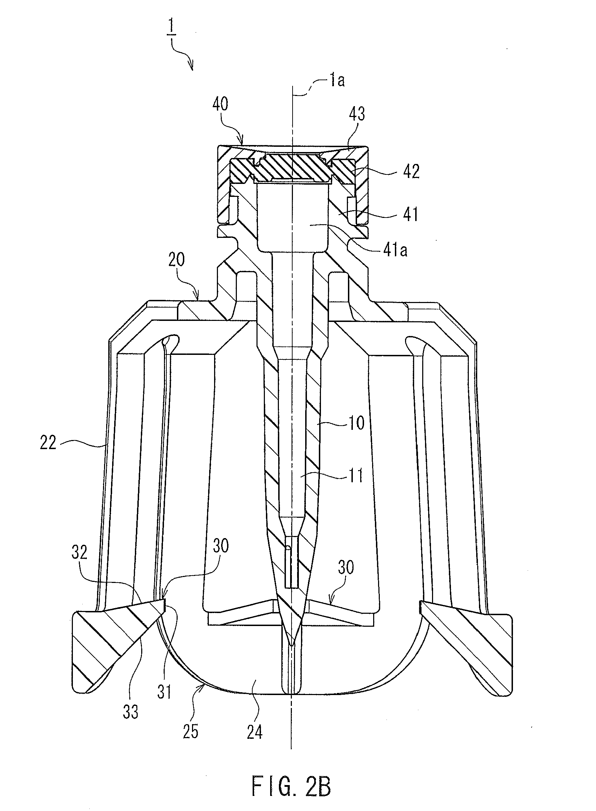

FIG. 1A is a perspective view showing a puncture needle adapter 1 observed from above according to one embodiment of the present invention, and FIG. 1B is a perspective view showing the puncture needle adapter 1 observed from below. FIG. 2A is a cross-sectional view showing the puncture needle adapter 1 taken along a plane in the up-down direction including the line 2A-2A in FIG. 1A, and FIG. 2B is a cross-sectional view showing the puncture needle adapter 1 taken along a plane in the up-down direction including the line 2B-2B in FIG. 1A. In FIG. 2A and FIG. 2B, the alternate long and short dash line 1a indicates the central axis of the puncture needle adapter 1. For convenience in the following description, the direction of the central axis 1a will be referred to as the "up-down direction" and the upper side of each of the drawings in FIG. 2A and FIG. 2B is regarded as "upper" of the puncture needle adapter 1, and the lower side of each of the drawings in FIG. 2A and FIG. 2B is regarded as "lower" of the puncture needle adapter 1. The direction parallel to the plane perpendicular to the central axis 1a is regarded as the "horizontal direction", and the direction of a straight line orthogonal to the central axis 1a is regarded as the "radial direction". Note that the "up-down direction", the "upper", the "lower" and the "horizontal direction" do not mean orientations during actual use of the puncture needle adapter 1.

The puncture needle adapter 1 includes a puncture needle 10 extending along the central axis 1a. The puncture needle 10 protrudes downward from the center of an approximately cross-shaped top plate 20, and it has a sharp tip at the lower end. Inside the puncture needle 10, a channel 11 where a liquid flows (for example, medical liquid) is formed. On the tapered outer circumferential face of the puncture needle 10 in the vicinity of the tip, two openings 12 communicating with the channel 11 are formed. The two openings 12 are provided symmetrically about the central axis 1a.

The top plate 20 includes four arms 21 extending approximately along the radial direction. The four arms 21 are arranged at equiangular intervals about the central axis 1a. As a result, the top plate 20 has an approximately cross shape. From the tip 21a of each of the arms 21, two elastic pieces 22 extend downward to oppose the puncture needle 10. The two elastic pieces 22 provided to a common arm 21 are divided by a slit 23. In the up-down direction, the slit 23 reaches the approximately same height as the tip 21a of the arm 21.

One of the two elastic pieces 22 extending from an arm 21 is joined at the tip (lower end) with the closer elastic piece 22 extending from the adjacent arm 21 so as to constitute an approximately U-shaped holding piece 25. The portion for joining (bridging) the tips of the two elastic pieces 22 constituting the holding piece 25 is referred to as a bridging portion 24 of the holding piece 25. In other words, the holding piece 25 includes two elastic pieces 22 and a bridging portion 24 that joins the tips of the two elastic pieces 22, and the base ends (upper end) of the two elastic pieces 22 are joined respectively with other arms 21 adjacent to each other. The thus constituted approximately U-shaped four holding pieces 25 are arranged with slits 23 therebetween at equiangular intervals about the central axis 1a so as to surround the puncture needle 10. On the surface of the bridging portion 24 opposing the puncture needle 10, an engaging claw 30 protruding toward the puncture needle 10 is provided. A lower edge 24a of the bridging portion 24 is shaped to have a smooth curve.

As shown in FIG. 1A, the top plate 20 has an approximately cross shape due to the four protruding arms 21, and adjacent arms 21 are joined with each other by an approximately U-shaped holding piece 25, thereby a large opening 26 surrounded by the top plate 20 and the holding piece 25 is formed. The puncture needle 10 can be observed from the outside of the puncture needle adapter 1 through the opening 26.

An elastic piece 22 is a thin strip whose main face (a surface having the largest area) opposes the puncture needle 10. The longitudinal direction of the elastic piece 22 is approximately parallel to the puncture needle 10. However, in the strict sense, as can be understood from FIG. 2A, the elastic piece 22 is inclined slightly relative to the central axis 1a such that the distance to the puncture needle 10 is increased as it goes downward (namely, the puncture needle adapter 1 as a whole widens toward the end). Such an elastic piece 22 can be bent and deformed elastically in the plane including the central axis 1a. Two elastic pieces 22 constituting the holding piece 25 undergo bending deformation, thereby the engaging claw 30 can be displaced in a direction away from the puncture needle 10.

FIG. 2B is a cross-sectional view showing the puncture needle adapter 1 taken along a plane passing the central axis 1a and an apex portion 31 of the engaging claw 30 located the closest to the puncture needle 10. As most evidently shown in this FIG. 2B, the engaging claw 30 includes an engaging face 32 opposing the top plate 20 and a slidable face 33 located lower than the apex portion 31. The engaging face 32 is inclined slightly to approach the top plate 20 with decreasing distance from the puncture needle 10 in the radial direction. Similarly, the slidable face 33 is inclined to approach the top plate 20 with decreasing distance from the puncture needle 10 in the radial direction. In comparison with the engaging face 32, the slidable face 33 has a larger inclination angle relative to the radial direction. The apex portion 31 is provided at a position where the engaging face 32 and the slidable face 33 intersect in a wedge shape.

At the center of the top plate 20, a connector 40 protrudes upward. As shown in FIG. 2A and FIG. 2B, the connector 40 includes a tubular portion 41 having an approximately cylindrical shape, a partition wall member (which may be referred to as a "septum") 42 provided on the upper end of the tubular portion 41, and a cap 43 covering the partition wall member 42. An inner cavity 41a inside the tubular portion 41 is in communication with a channel 11 inside the puncture needle 10. The partition wall member 42 is a sheet made of an elastic material such as rubber, having a circular shape in a plan view. At the center of the partition wall member 42, a straight slit (incision) 42a passing through the partition wall member 42 in the up-down direction is formed. The partition wall member 42 is placed on the upper end of the tubular portion 41, and the partition wall member 42 is covered from above with the cap 43. An engaging protrusion 41p protruding from the outer circumferential face of the tubular portion 41 fits into an engaging hole 43h passing through the surrounding wall of the cap 43, and thus the engaging protrusion 41p and the engaging hole 43h engage with each other (see FIG. 1A and FIG. 1B). The partition wall member 42 is sandwiched in the up-down direction by the tubular portion 41 and the cap 43. The slit 42a of the partition wall member 42 is exposed within the opening 43a formed on the upper face of the cap 43 (see FIG. 1A). When a tubular male luer (not shown) that does not have a sharp tip is inserted into the slit 42a of the partition wall member 42, the partition wall member 42 is elastically deformed, thereby the inner cavity 41a inside the tubular portion 41 and the male luer communicate with each other. When the male luer is pulled out from the partition wall member 42, the partition wall member 42 immediately returns its initial state, and the slit 42a is closed in a liquid-tight state. In this manner, the partition wall member 42 functions as a self-closing valve disc. Such a self-closing connector 40 is called also as "needleless port".

It is preferable that the components of the puncture needle adapter 1 of the present embodiment, i.e., the puncture needle 10, the top plate 20, the holding piece 25, the engaging claw 30 and the tubular portion 41 are formed integrally by eject-molding a resin material. Though there is no particular limitation on the applicable resin material, the examples include polyethylene, polypropylene, polycarbonate, styrene-ethylene, polyethylene terephthalate, polybutylene terephthalate, and butylene-styrene block copolymer. From the viewpoint of application in a medical field and repeated elastic bending deformation of the elastic pieces 22, polyolefin-based resins such as polyethylene and polypropylene are preferred. Though there is no particular limitation on the material of the cap 43, a hard material is preferred, and for example, polycarbonate, polypropylene, polyacetal, polyamide, rigid polyvinyl chloride, polyethylene and the like can be used. Though there is no particular limitation on the material of the partition wall member 42, a soft material is preferred, and for example, rubber materials such as isoprene rubber, silicone rubber, butyl rubber, a thermoplastic elastomer and the like can be used.

Hereinafter, method of using the thus constituted puncture needle adapter 1 of the present embodiment will be explained.

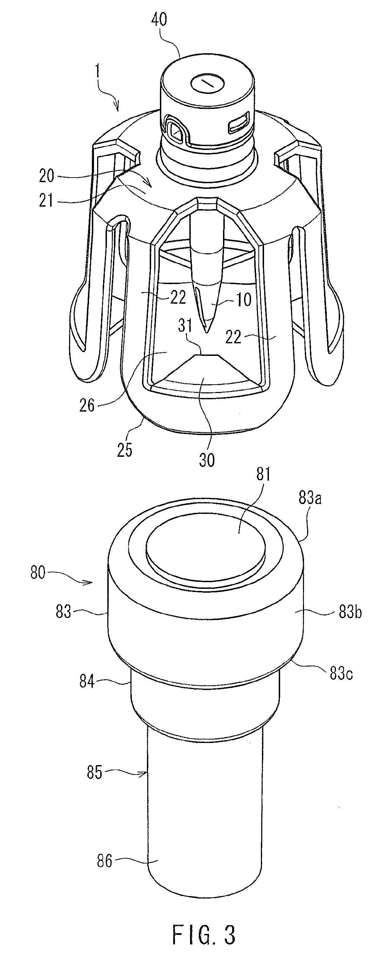

First, as shown in FIG. 3, the puncture needle adapter 1 is arranged to oppose a female connector 80. The female connector 80 is a so-called a "rubber plug" where a columnar rubber member 81 is allowed to fit into the central opening of a flange 83 having a cylindrical shape so as to seal the opening in a liquid-tight state. A constitution similar to that of the female connector 80 is provided also to a vial for example. The flange 83 is provided to one end of a connector body 85 having a cylindrical shape, and the other end 86 of the connector body 85 is connected to a drug solution container (not shown) for storing a drug solution. The drug solution container is not limited in particular for the constitution, and it may be any arbitrary container such as a bag-like container that does not have shape retention, or bottle or box-like container that has shape retention but can be deformed easily when applied with an external force. The connector body 85 may be provided integrally as one part to the drug solution container.

The outer circumferential face 83b of the flange 83 is a cylindrical face. The flange 83 has an outer diameter greater than that of a portion (narrow portion) 84 directly below the flange 83 of the connecter body 85, and it expands in the radial direction. Therefore, at an lower edge 83c of the outer circumferential face 83b of the flange 83, a step is formed due to the difference in the outer diameter between the outer circumferential face 83b and the narrow portion 84 located below.

Starting from the state as shown in FIG. 3, the tip of the puncture needle 10 of the puncture needle adapter 1 is brought into contact with the rubber member 81, and further pressed into the rubber member 81. Before, after or approximately the same time the puncture needle 10 enters into the rubber member 81, the slidable face 33 (see FIG. 1B, FIG. 2A and FIG. 2B) of the engaging claw 30 of the holding piece 25 comes into contact with the upper outer circumferential edge (upper edge) 83a of the flange 83. With the increase in the depth of insertion of the puncture needle 10 relative to the rubber member 81, the upper edge 83a slides on the slidable face 33, the elastic pieces 22 of the holding piece 25 undergo elastic bending deformation, thereby the engaging claw 30 is displaced in the direction away from the puncture needle 10 (outward). The apex portion 31 of the engaging claw 30 surmounts the upper edge 83a, and then slides on the outer circumferential face 83b of the flange 83 downward. When the apex portion 31 of the engaging claw 30 reaches the lower edge 83c of the outer circumferential face 83b, the elastic pieces 22 of the holding piece 25 undergo elastic restoration, and the apex portion 31 fits into the narrow portion 84 located below the flange 83.

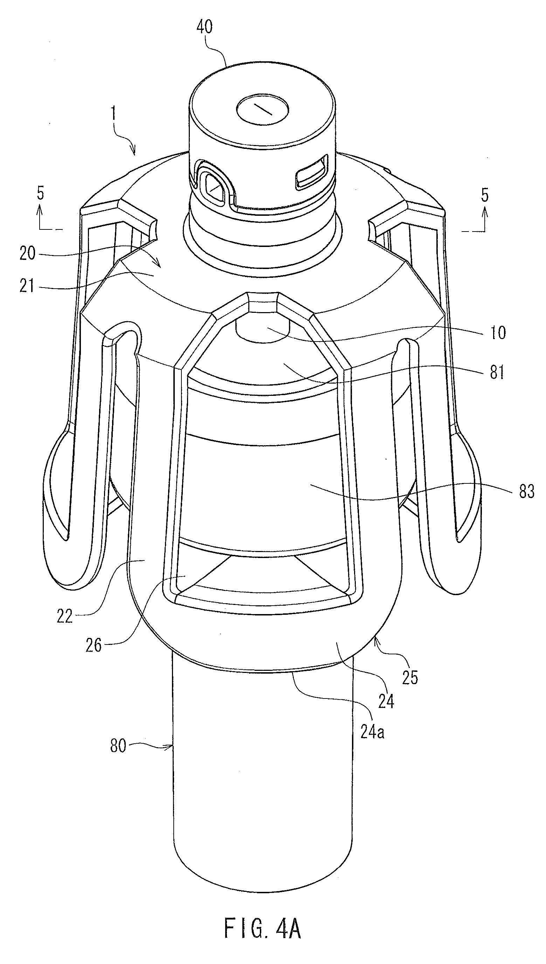

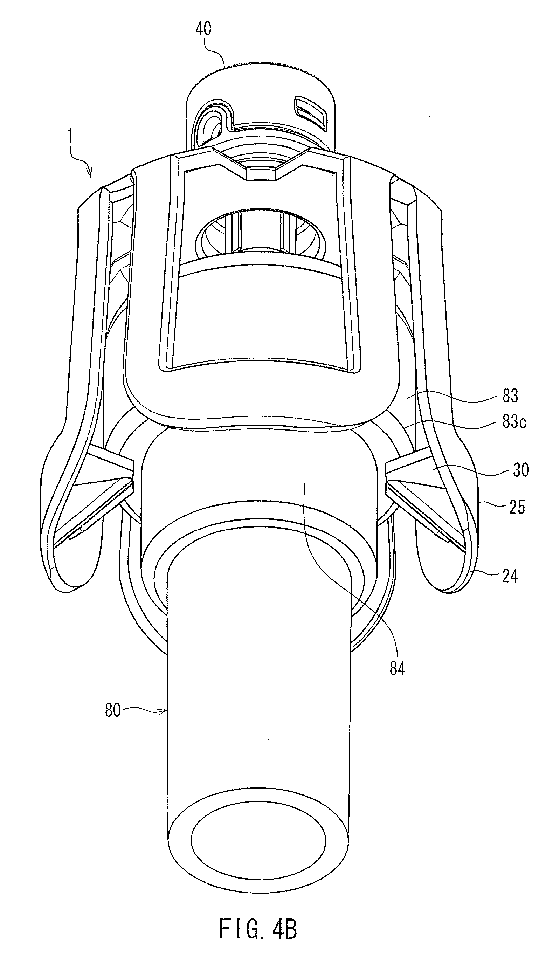

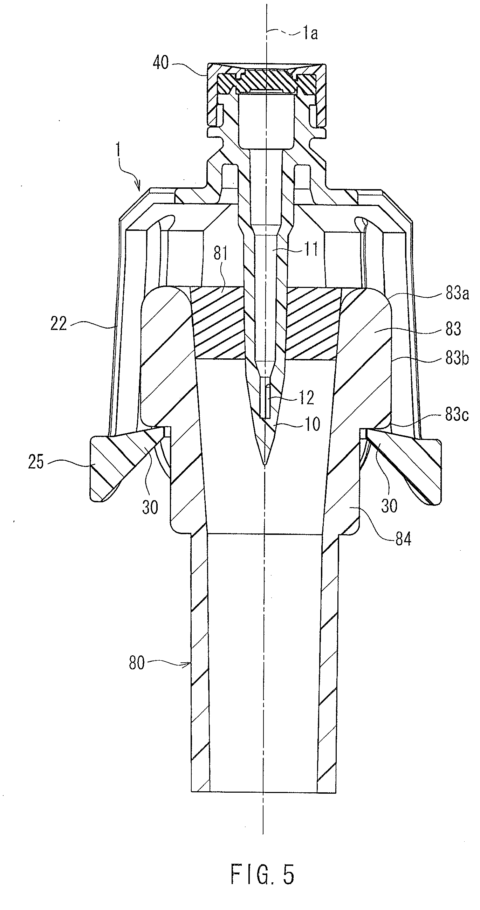

In this manner, as shown in FIG. 4A, FIG. 4B and FIG. 5, the puncture needle adapter 1 can be connected to the female connector 80. The cross section of FIG. 5 passes the central axis 1a and the apex portion 31 of the engaging claw 30.

As shown in FIG. 5, the puncture needle 10 passes through the rubber member 81, and the opening 12 formed in the vicinity of the tip of the puncture needle 10 is exposed below the rubber member 81. Thereby, the channel 11 of the puncture needle 10 and the female connector 80 communicate with each other.

The engaging claw 30 engages with the lower edge 83c of the flange 83. Therefore, even when an external force (e.g., a tensile force in the direction for separating the puncture needle adapter 1 from the female connector 80) or vibration is applied to the puncture needle adapter 1 or the female connector 80, the puncture needle 10 is prevented from unintentionally coming out of the rubber member 81.

The narrow and thin elastic piece 22 extending from the tip 21a of each arm 21 undergoes elastic bending deformation according to the outer diameter of the flange 83. Therefore, the range of outer diameter of the flange 83 to which the puncture needle adapter 1 of the present invention can be connected is relatively wide.

Since the range for allowing elastic bending formation of the elastic piece 22 is increased when the upper end position of the slit 23 (see FIG. 1A) is higher, the upper limit for the outer diameter of the connectable flange 83 is enlarged. When the slit 23 reaches the tip 21a of the arm 21 or the vicinity as in the present embodiment, the range to allow the elastic bending deformation of the elastic piece 22 is maximized, which is advantageous to enlarge the range in the outer diameter of the flange 83 (in particular, the upper limit) to which the puncture needle adapter 1 can be connected.

However, if the slit 23 reaches an arm 21 approximately parallel in the horizontal direction, the strength of the arm 21 deteriorates. If the strength of the arm 21 is low, when the upper edge 83a of the flange 83 comes into contact with the slidable face 33 of the engaging claw 30 during the process of connecting the puncture needle adapter 1 to the female connector 80, the elastic pieces 22 do not undergo bending deformation but the arm 21 undergoes bending deformation upward by the upward force applied by the upper edge 83a. In this case, it is impossible to engage the engaging claw 30 with the lower edge 83c of the flange 83. Therefore, it is preferable that the slit 23 does not reach the arm 21 that extends substantially along the horizontal direction.

The base ends of the two elastic pieces 22 constituting the holding piece 25 are connected to different arms 21. The main faces of the two elastic pieces 22 constituting the holding piece 25 do not form an identical plane but they oppose respectively the puncture needle 10. Therefore, when the engaging claw 30 is displaced in a direction away from the puncture needle 10, the elastic pieces 22 undergo bending deformation with a slight distortion. In this constitution, the fracture strength of the elastic pieces 22 against the bending deformation is improved in comparison with a constitution that the elastic pieces 22 simply undergo bending deformation without distortion. This is advantageous in enlarging the range of the elastic bending deformation.

Since the holding piece 25 has an approximately U-shape, as can be understood from FIG. 3 and FIG. 4A, through the large opening 26 surrounded by the holding piece 25 and the arm 21 of the top plate 20, the puncture needle 10 and the rubber member 81, and furthermore the state where the puncture needle 10 is stuck into the rubber member 81 can be visually observed easily. This is advantageous in conducting a further reliable puncture operation.

As can be understood from FIG. 5, when the outer diameter of the flange 83 is larger, the displacement amount of the engaging claw 30 outward in the radial direction at the time of connecting the puncture needle adapter 1 to the female connector 80 is increased. When the engaging claw 30 is displaced outward in the radial direction, the engaging claw 30 swings and its posture (orientation) changes. As mentioned above, in an initial state (unloaded state) where the elastic pieces 22 do not undergo bending deformation, the engaging face 33 of the engaging claw 30 is inclined such that the apex portion 31 side is located closer to the top plate 20. Due to this constitution, even when the engaging claw 30 is displaced outward in the radial direction, it is possible to keep the apex portion 31 at a position higher than the engaging face 33. Therefore, since the engaging face 33 is inclined as described above, even in a case where the flange 83 has a large outer diameter, the resistance to cancellation of engagement between the engaging claw 30 and the flange 83 can be ensured when the puncture needle adapter 1 and the female connector 80 connected to each other as shown in FIG. 5 are applied with a tensile force in a direction for separating these components. As a result, even when the flange 83 of the female connector 80 has a large outer diameter, the chance that the puncture needle 10 unintentionally comes out of the rubber member 81 can be decreased further. However, in the initial state where the elastic pieces 22 do not undergo bending deformation (unloaded state), there is no necessity that the engaging face 33 be inclined as described above, but it may be parallel to the horizontal plane, for example.

On the side opposite to the top plate 20 relative to the apex portion 31, the engaging claw 30 has a slidable face 33 that is inclined to approach the top plate 10 with decreasing distance from the puncture needle 10. Therefore, as described above, simultaneously with the action of pressing the puncture needle 10 into the rubber member 81, the engaging claw 30 comes into contact with the upper edge 83a of the flange 83 and is displaced. Therefore, any special operations for engaging the engaging claw 30 with the flange 83 is not necessary, and thus the operation for connecting to the female connector 80 can be conducted easily.

The elastic pieces 22 are inclined to be widened toward the end so as to be separated from the puncture needle 10 with increasing distance from the top plate 20. Since the elastic pieces 22 are inclined in this manner, when the upper edge 83a of the flange 83 applies an upward force to the slidable face 33 of the engaging claw 30 during a process of connecting the puncture needle adapter 1 to the female connector 80, the elastic pieces 22 can be bent and deformed easily without bending and deforming upward the arm 21. Therefore, the pressing force applied to the puncture needle adapter 1 toward the female connector 80, which is required to connect the puncture needle adapter 1 to the female connector 80, can be decreased. However, the elastic pieces 22 are not required to be inclined as described above, but they may be parallel to the central axis 1a for example.

Since the edge 24a of the bridging portion 24 of the holding piece 25 is formed with a smooth curve, even when a finger of an operator touches the edge 24a, the operator does not feel pain or injure his/her finger. Therefore, for example, as shown in FIG. 4A, FIG. 4B and FIG. 5, the operator can conduct an operation of separating the connected puncture needle adapter 1 and the female connector 80, in particular, an operation of disengaging the engaging claw 30 and the flange 80 easily by putting his/her finger on the bridging portion 24 of the holding piece 25.

The connector 40 provided on the upper face of the top plate 20 is a self-closing connector. Accordingly, when the male luer connected to the connector 40 is pulled out of the connector 40, the slit 42a in the partition wall member 42 is closed immediately. As a result, the chance that a dangerous drug solution leaks out to the outside can be decreased.

The Embodiments described above are merely illustrative examples. The present invention is not limited to the Embodiments described above, and can be modified as appropriate.

In the Embodiments described above, only one channel 11 where a liquid flows is formed inside the puncture needle 10. In addition to the channel where a liquid flows, a channel where a gas flows may be formed to prevent a negative pressure inside the container.

The constitution of the connector 40 formed on the upper face of the top plate 20 is not limited to the needleless port illustrated in Embodiments described above. For example, it may be a well-known connector including a tapered face. Alternatively, onto the upper face of the top plate 20, a flexible tube of an administration set may be connected directly not via a detachable connector.

The constitution of the container including a female connector to be punctured with the puncture needle 10 can be determined arbitrarily. The container may be a deformable container, or a container such as a vial that is substantially not deformable.

The liquid that flows in the channel of the puncture needle 10 may be a drug solution or may be an arbitrary liquid other than a drug solution.

### Industrial Applicability

Though there is no particular limitation on the field for application, the present invention can be used preferably as a puncture needle adapter to be connected to a rubber plug of a female connector of a container that stores a liquid (in particular, drug solution).

### Explanation of Letters and Numerals

- 1: puncture needle adapter
- 10: puncture needle
- 11: channel
- 20: top plate
- 21: arm
- 21a: tip of arm
- 22: elastic piece
- 23: slit
- 24: bridging portion
- 24a: edge of bridging portion
- 25: holding piece
- 26: opening
- 30: engaging claw
- 31: apex portion
- 32: engaging face
- 33: slidable face
- 40: connector
- 80: female connector

## Claims

1. A puncture needle adapter comprising a puncture needle and an engaging claw to be engaged with a female connector when the puncture needle is stuck into the female connector,
wherein the puncture needle adapter comprises further a top plate from which four arms protrude to form an approximately cross shape,
the puncture needle protrudes from the center of the top plate,
two elastic pieces divided by a slit extend from the tip of each of the four arms toward the same side as the puncture needle so as to oppose the puncture needle,
the tips of two of the elastic pieces extending respectively from two adjacent arms are joined with each other via a bridging portion so as to form an approximately U-shaped holding piece,
the engaging claw is formed at the bridging portion of the holding piece so as to protrude toward the puncture needle, and
the engaging claw is displaceable in a direction away from the puncture needle by elastic deformation of the two elastic pieces constituting the holding piece.

2. The puncture needle adapter according to claim 1, wherein an engaging face of the engaging claw to oppose the top plate is inclined to approach the top plate with decreasing distance from the puncture needle.

3. The puncture needle adapter according to claim 1 or 2, wherein the elastic pieces are inclined to be separated from the puncture needle with increasing distance from the top plate.

4. The puncture needle adapter according to any one of claims 1 to 3, wherein the slit reaches the tip of the arm or the vicinity.

5. The puncture needle adapter according to any one of claims 1 to 4, wherein an edge of the bridging portion of the holding piece at the side opposite to the top plate is formed of a smooth curve.

6. The puncture needle adapter according to any one of claims 1 to 5, wherein the engaging claw comprises a slidable face at the side opposite to the top plate, inclined to approach the top plate with decreasing distance from the puncture needle.

7. The puncture needle adapter according to any one of claims 1 to 6, wherein a self-closing connector communicating with the puncture needle is provided on a surface of the top plate opposite to the puncture needle.
